# EUROPÄISCHE PATENTSCHRIFT

(11) **EP 3 740 319 B1**
(45) Veröffentlichungstag und Bekanntmachung des Hinweises auf die Patenterteilung: **03.04.2024**
(21) Anmeldenummer: 18703490.5
(22) Anmeldetag: 16.01.2018
(51) Int. Cl.: B03D 1/01, C07C 213/08

(54) **ESTERQUATS ZUR FLOTATION VON NICHT-SULFIDISCHEN MINERALIEN UND ERZEN UND VERFAHREN**
ESTERQUATS FOR THE FLOTATION OF NON-SULFIDIC MINERALS AND ORES, AND METHOD
ESTERQUATS POUR LA FLOTTATION DE MINÉRAUX ET MINERAIS NON SULFURÉS ET PROCÉDÉ DE FLOTTATION

(43) Veröffentlichungstag der Anmeldung: 25.11.2020
(73) Patentinhaber: Clariant International Ltd, 4132 Muttenz (CH)
(72) Erfinder: ARNDT, Matthias, 63128 Mannheim (DE); PEDAIN, Klaus-Ulrich, 63128 Dietzenbach-Steinberg (DE); MUELLER, Pia, 79588 Efringen-Kirchen (DE); SOLDUGA RAMIREZ, Gemma, 68167 Mannheim (DE); SCHUNK, Yves, 65929 Frankfurt (DE); KOCHAN, Jozef, 65719 Hofheim (DE)
(74) Vertreter: Clariant Produkte (Deutschland) GmbH
(86) Internationale Anmeldenummer: PCT/EP2018/050914
(87) Internationale Veröffentlichungsnummer: WO 2019/141343

(56) Entgegenhaltungen:
- EP-A1- 1 949 964
- WO-A1-2008/089906
- C. L. Noller: "Polycarbonsäuren", Lehrbuch der Organischen Chemie, 1. Januar 1960 (1960-01-01), XP055477296, Berlin, Heidelberg Gefunden im Internet: URL:https://www.springer.com/de/book/97836 42873256 [gefunden am 2018-05-22]

## Beschreibung

Die vorliegende Erfindung betrifft neue Esterquats, erhältlich, indem man Di- oder Trialkanolamine mit Fettsäuren und Polycarbonsäuren umsetzt und die so erhaltenen Ester - gegebenenfalls nach Alkoxylierung - quaterniert. Die Erfindung bezieht sich weiterhin auf ein Verfahren zur Schaumflotation von nicht-sulfidischen Mineralien und Erzen und insbesondere die Verwendung der Esterquats als Kollektor in einem Schaumflotationsverfahren.

Flotation ist eine Trenntechnik, die üblicherweise bei der Aufbereitung von Mineralien verwendet wird. Dabei werden die Roherze in Wertstoff und Gangart getrennt. Nicht-sulfidische Mineralien und Erze im Rahmen der vorliegenden Erfindung umfassen beispielsweise Calcit, Apatit, Fluorit, Scheelit, Baryt, Eisenoxide und andere Metalloxide, beispielsweise die Oxide von Titan und Zirkonium, sowie bestimmte Silikate und Alumosilikate.

Bei flotationsbasierten Aufbereitungsprozessen wird das Mineral oder Erz zuerst durch Trocken- oder vorzugsweise Nassmahlung zerkleinert und in Wasser suspendiert. Anschließend wird der Kollektor, häufig in Verbindung mit Schäumern und anderen Hilfsreagenzien, wie Drückern oder Aktivatoren, dem Erz-WasserGemisch zugefügt, um den Wertstoff aus der unerwünschten Gangart des Erzes zu trennen. Nach einer bestimmten Einwirkzeit der Reagenzien (Konditionierung) wird Luft in die Suspension eingeleitet, die aufgrund der Zellgeometrie feinverteilt wird und an der Oberfläche der Flotationszelle einen Schaum erzeugt.

Der Kollektor hydrophobiert die Oberfläche der Mineralien, so dass diese an den Luftbläschen anhaften, wenn Luftblase und Partikel aufeinandertreffen. Die Kollektoren hydrophobieren die Mineralien selektiv, sodass im Falle einer direkten Flotation die wertvollen Mineralien zur Oberfläche aufschwemmen und dass sich im Falle einer reversen Flotation die Partikel der Gangart an der Oberfläche sammeln. Der feststoffhaltige Schaum wird an der Zelloberfläche abgestreift und weitertransportiert. Das Ziel der Flotation ist es, das wertvolle Material der Mineralien und Erze mit möglichst hoher Ausbeute von der Gangart zu trennen, wobei gleichzeitig eine hohe Anreicherung des wertvollen Minerals erreicht werden soll.

Bei der nicht-sulfidischen Flotation, beispielsweise bei der reversen CalcitFlotation werden insbesondere anionische, kationische und ampholytische Tenside als Kollektoren eingesetzt, die meist auch eine schäumende Wirkung haben, sodass auf eine weitere Zugabe eines Schäumers verzichtet werden kann. Calcit ist ein wichtiger Füllstoff in der Papierindustrie, da man damit den Weißheitsgrad und die Transparenz des Papiers einstellen kann. Calcit Mineralien werden jedoch oft durch Silikate, Eisenoxide, etc. begleitet, die die Reinheit negativ beeinflussen und somit entfernt werden müssen.

Als Stand der Technik wird WO-2008/089906 angesehen, in dem die Verwendung von polymeren Esterquats als Kollektoren für Silikate und Magnesiumsalze bei der nicht-sulfidischen Flotation als besonders wirksam beschrieben sind.

DE-102008056338 lehrt die Verwendung einer Zusammensetzung aus
A) mindestens einer quaternären Ammoniumverbindung, die mindestens einen, an das Ammoniumstickstoffatom gebundenen, gegebenenfalls Heteroatome enthaltenden, organischen Rest mit 1 bis 36 Kohlenstoffatomen enthält, und
B) mindestens einem Aminalkoxylatester der Formel (1) oder einem Salz davon 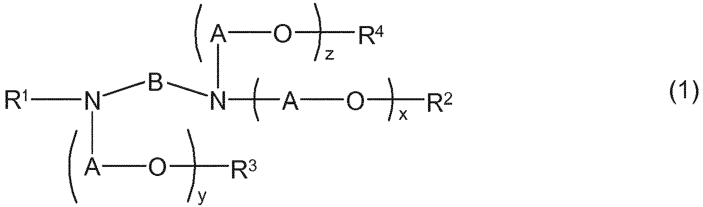 worin
   - A, B: unabhängig voneinander ein C₂- bis C₅-Alkylenrest
   - R¹: ein C₈- bis C₂₄-Alkylrest oder -Alkenylrest
   - R², R³, R⁴: unabhängig voneinander H oder ein C₈- bis C₂₄-Acylrest, mit der Maßgabe, dass mindestens einer der Reste R², R³ oder R⁴ für einen C₈₋ bis C₂₄-Acylrest steht
   - x, y, z: unabhängig voneinander eine ganze Zahl von 0 bis 50 mit der Maßgabe, dass x + y + z eine ganze Zahl von 1 bis 100 ist, bedeuten,
   in Mengen von 10 bis 5000 g/Tonne Erz als Sammler in der Silikatflotation.

Die Aufgabe der vorliegenden Erfindung ist es, einen verbesserten Kollektor für verschiedene Flotationsverfahren bereitzustellen, mit dem es möglich ist bei gleicher Kollektormenge eine bessere Selektivität bei gleichbleibend hoher Ausbeute zu erreichen. Insbesondere sollte der Kollektor für die reverse CalcitFlotation geeignet sein, und ein reineres Calcit bei möglichst geringem Verlust ergeben.

Überraschend wurde gefunden, dass Esterquats, die man erhält, indem man Di- oder Trialkanolamine mit Fettsäuren und Polycarbonsäuren umsetzt und die resultierenden Ester - gegebenenfalls nach Alkoxylierung - in an sich bekannter Weise quaterniert, einen verbesserten Kollektor für verschiedene Flotationsverfahren darstellen. Die neuen Esterquats haben sich, insbesondere bei der reversen Calcitflotation, überraschenderweise als noch wirksamer herausgestellt und weisen darüber hinaus eine sehr gute biologische Abbaubarkeit auf, die besonders für die Calcithersteller eine große Rolle spielt.

Gegenstand vorliegender Erfindung sind daher Esterquats, dadurch erhältlich, indem man Di- oder Trialkanolamine mit einer Mischung aus Fettsäuren und Polycarbonsäuren umsetzt, und die so erhaltenen Ester anschließend mit einem Alkylierungsmittel quaterniert, wobei die Di- oder Trialkanolamine der Formel (I) entsprechen 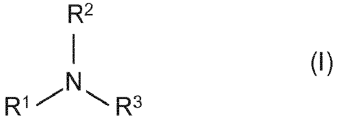 worin
- R¹ und R²: unabhängig voneinander für Hydroxyalkylreste mit 1 bis 20 Kohlenstoffatomen, Hydroxyalkenylreste mit 2 bis 20 Kohlenstoffatomen und 1, 2, oder 3 Doppelbindungen, oder Anlagerungsprodukte von 1 bis 20 und vorzugsweise 2 bis 5 Mol Ethylenoxid an einen Hydroxyethylrest stehen, und
- R³: für Wasserstoff, einen Alkylrest mit 1 bis 20 Kohlenstoffatomen, einen Alkenylrest mit 2 bis 20 Kohlenstoffatomen und 1, 2, oder 3 Doppelbindungen, einen Hydroxyalkylrest mit 1 bis 20 Kohlenstoffatomen, einen Hydroxyalkenylrest mit 2 bis 20 Kohlenstoffatomen und 1, 2, oder 3 Doppelbindungen, oder Anlagerungsprodukte von 1 bis 20 und vorzugsweise 2 bis 5 Mol Ethylenoxid an einen Hydroxyethylrest steht, und wobei
die Fettsäuren der Formel (II) entsprechen,

R⁴-COOH (II)

worin
- R⁴: für einen aliphatischen, linearen oder verzweigten Kohlenwasserstoffrest mit 5 bis 29 Kohlenstoffatomen und 0, 1, 2 oder 3 Doppelbindungen steht, und wobei
die Polycarbonsäuren der Formel (III) entsprechen 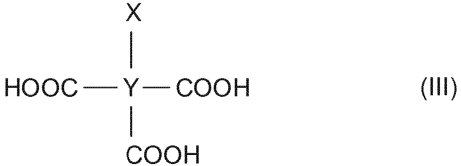 worin
- Y: für ein Kohlenstoffatom oder eine gesättigte oder ungesättigte, aliphatische Kohlenwasserstoffgruppe mit 2 bis 5 Kohlenstoffatomen steht, welche gegebenenfalls eine oder mehrere Hydroxylgruppen trägt, und
- X: für Wasserstoff, eine Hydroxy-, oder eine Carbonsäuregruppe stehen.

Ein weiterer Gegenstand der vorliegenden Erfindung betrifft die Verwendung der erfindungsgemäßen Esterquats als Kollektor in der Flotation von nicht-sulfidischen Mineralien oder Erzen.

Ein weiterer Gegenstand der vorliegenden Erfindung betrifft ein Verfahren zur Flotation von nicht-sulfidischen Mineralien oder Erzen, bei dem das erfindungsgemäße Esterquat als Kollektor zugegeben wird.

Ein weiterer Gegenstand der Erfindung betrifft ein Verfahren zur Herstellung von Esterquats, bei dem man Di- oder Trialkanolamine mit einer Mischung aus Fettsäuren und Polycarbonsäuren umsetzt und die resultierenden Ester gegebenenfalls alkoxyliert und anschließend in bekannter Weise quaterniert.

Im Flotationsverfahren wird das Roherz zuerst vermahlen und anschließend mit Wasser zu einer Suspension vermischt wird. Diesem Gemisch werden der erfindungsgemäße Kollektor und bei Bedarf weitere Reagenzien hinzugegeben und Luft eingeblasen, wodurch sich eine Schaumschicht bildet, in der die hydrophoben Mineralpartikel aufgeschwemmt wurden.

Überraschenderweise wurde beobachtet, dass die neuen Esterquats äußerst wirksame Kollektoren für die Flotation von nicht-sulfidischen Mineralien und Erze sind, insbesondere im Hinblick auf die Anwesenheit von Silikaten und / oder Magnesiumsalze in den Mineralien oder Erzen. Die Kollektoren nach der vorliegenden Erfindung sind effektiver im Vergleich zu den herkömmlichen Esterquats mit Mono- und Polycarbonsäuren, während sie einen sehr hohen Grad an Bioabbaubarkeit aufweisen. Insbesondere haben sich die Produkte für die Trennung durch Schaumflotation von silikatischen Mineralien aus Calcit als sehr nützlich erwiesen.

Durch Veresterung mit einer Mischung von Fettsäuren und Polycarbonsäuren werden neue Esterquats der Formel (1) erhalten, die sich überraschenderweise gegenüber Produkten des Stands der Technik nicht nur durch eine besonders gute ökologische Verträglichkeit, sondern auch durch hervorragende Ausbeuten bei der Flotation auszeichnen.

Die Di- oder Trialkanolamine der Formel (I) umfassen als Reste R¹ und R² unabhängig voneinander vorzugsweise Hydroxyalkylreste mit 2 bis 5 Kohlenstoffatomen, oder Hydroxyalkenylreste mit 2 bis 5 Kohlenstoffatomen und 1, 2 oder 3 Doppelbindungen, oder Anlagerungsprodukte von 2 bis 5 Mol Ethylenoxid an einen Hydroxyethylrest.

R³ steht vorzugsweise für Alkylreste mit 1 bis 20 Kohlenstoffatomen, Alkenylreste mit 2 bis 20 Kohlenstoffatomen, Hydroxyalkylreste mit 2 bis 5 Kohlenstoffatomen oder Hydroxyalkenylreste mit 2 bis 5 Kohlenstoffatomen und 1, 2, oder 3 Doppelbindungen.

Bevorzugte Di- oder Trialkanolamine der Formel (I) sind Methyldiethanolamin (MDA), Diethanolamin (DEA), diethoxylierte Oleylamine und Triethanolamin (TEA).

In einer besonders bevorzugten Ausführungsform der vorliegenden Erfindung wird Triethanolamin verwendet.

In den Fettsäuren der Formel (II) steht R⁴ vorzugsweise für einen aliphatischen, linearen oder verzweigten Rest mit 7 bis 21 Kohlenstoffatomen und 0, 1, 2 oder 3 Doppelbindungen.

Bevorzugte Fettsäuren sind aliphatische Carbonsäuren ausgewählt aus der Gruppe bestehend aus Caprylsäure, , Caprinsäure, Laurinsäure, Undecylensäure, Isotridecansäure, Myristinsäure, Myristoleinsäure, Palmitinsäure, Palmitoleinsäure, Stearinsäure, Isostearinsäure, Ölsäure, Petroselinsäure, Elaidinsäure, Petroselinsäure, Linolsäure, Linolensäure, Elaeostearinsäure, Arachinsäure, Gadoleinsäure, Behensäure, Erucasäure und Cetoleinsäure sowie deren technische Mischungen. Diese Carbonsäuren entstehen beispielsweise bei der Druckspaltung von natürlichen Fetten und Ölen, bei der Reduktion von Aldehyden aus der Roelen'schen Oxosynthese oder der Dimerisierung von ungesättigten Fettsäuren. Die genannten Fettsäuren können, falls ungesättigt, in ihrer hydrierten oder teilweise hydrierten Form verwendet werden.

Ebenfalls bevorzugt sind technische Fettsäuregemische, die Fettsäuren mit 12 bis 18 Kohlenstoffatomen, beispielsweise Kokosöl-, Palmöl-, Palmkernöl- oder Talgfettsäuren, enthalten.

Die Polycarbonsäure der Formel (III) umfasst vorzugsweise als Rest Y eine gesättigte oder ungesättigte aliphatische Kohlenwasserstoffgruppe mit 3 oder 4 Kohlenstoffatomen, welche gegebenenfalls eine oder mehrere Hydroxylgruppen tragen kann.

Bevorzugte Polycarbonsäuren sind Propan-1,2,3-tricarbonsäure, Aconitsäure, Isozitronensäure und insbesondere Zitronensäure.

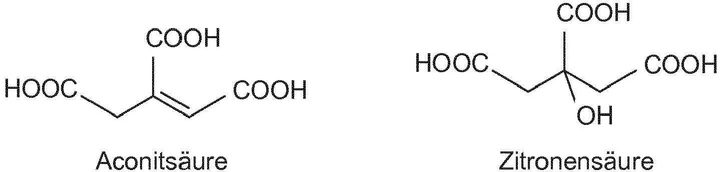

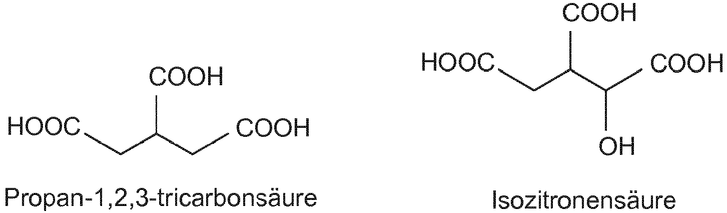

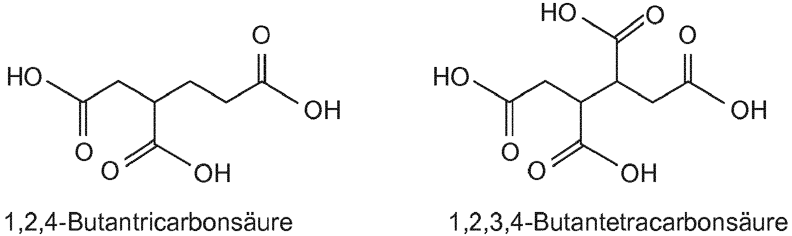

Die Fettsäuren und die Polycarbonsäuren können vorzugsweise im molaren Verhältnis von Fettsäure zu Polycarbonsäure = 1:10 bis 10:1 eigesetzt werden. Es hat sich als besonders vorteilhaft erwiesen, ein Molverhältnis von 1:1 bis 8:1 einzustellen.

Die Di- oder Trialkanolamine einerseits und die Säuren - also Fettsäuren und Polycarbonsäuren zusammengenommen - andererseits können bevorzugt im molaren Verhältnis von Di- oder Trialkanolamine zu Säuren von 1:0,25 bis 1:3,0, insbesondere im Molverhältnis von 1:0,6 bis 1:1,5 eingesetzt werden.

Die Veresterung kann in an sich bekannter Weise durchgeführt werden, beispielsweise wie in WO 91/01295 beschrieben. In einer vorteilhaften Ausführungsform wird die Reaktion bei Temperaturen zwischen 120 °C und 220 °C und insbesondere von 140 °C bis 200 °C und Drücken von 0,01 bis 1 bar durchgeführt. Um die Veresterungsreaktion zu beschleunigen kann ein Katalysator eingesetzt werden. Geeignete Katalysatoren sind Säuren, vorzugsweise hypophosphorige und phosphorige Säuren und deren Alkalisalze, vorzugsweise Natriumhypophosphit, die in Mengen von 0,001 bis 0,5 Gewichts-% verwendet werden können, und vorzugsweise in Mengen von 0,005 bis 0,15 Gewichts-% bezogen auf die Einsatzstoffe.

Es ist möglich, zur Veresterung Mischungen der Fettsäuren und Polycarbonsäuren einzusetzen, oder aber die Veresterung mit den beiden Komponenten nacheinander durchzuführen.

Im Hinblick auf eine besonders hohe Farbqualität und -stabilität hat sich die Mitverwendung von Alkali- und/oder Erdalkaliborhydriden, wie beispielsweise Kalium-, Magnesium-, und insbesondere Natriumborhydrid in der Veresterung als vorteilhaft erwiesen. Diese Verbindungen setzt man üblicherweise in Mengen von 50 bis 1000 ppm und insbesondere 100 bis 500 ppm - wieder bezogen auf die Einsatzstoffe - ein wie beschrieben in der Anmeldung DE-C1-44 09 322.

Auch eine Behandlung der Esters mit Peroxidverbindungen oder eine Mischung aus Peroxidverbindungen und Alkaliboranaten vor der Quaternierung führt zu hoher Farbqualität und -stabilität. Als Peroxidverbindungen kommt neben Percarbonsäuren und Percarbonaten vorzugsweise Wasserstoffperoxid in Betracht. Unter Alkaliboranaten sind Lithium-, Kalium- und vorzugsweise Natriumboranat zu verstehen. Vorteilhafterweise werden die Peroxidverbindungen und die Alkaliboranate jeweils in Mengen von 0,005 bis 0,1, vorzugsweise 0,03 bis 0,06 Gew.-% - bezogen auf die Veresterungsprodukte - eingesetzt, wie in DE 43 08 792 beschrieben.

Zur Herstellung von polyalkylenoxidhaltigen Esterquats kann man nach zwei Alternativen verfahren. Zum einen kann man ethoxylierte Alkanolamine einsetzen. Dies hat den Vorteil, dass die Alkylenoxidverteilung im später resultierenden Esterquat bezüglich der OH-Gruppen des Amins annähernd gleich ist. Nachteilig ist jedoch, dass die Veresterung aus sterischen Gründen schwieriger wird. Die bevorzugte Methode besteht daher darin, den Ester vor der Quaternierung zu alkoxylieren. Dies kann in an sich bekannter Weise geschehen, d. h. in Anwesenheit basischer Katalysatoren und bei erhöhten Temperaturen. Als Katalysator kommen beispielsweise Alkali- und Erdalkalihydroxide und -alkoholate, vorzugsweise Natriumhydroxid und insbesondere Natriummethanolat in Betracht. Die Einsatzmenge liegt üblicherweise bei 0,5 bis 5 und vorzugsweise 1 bis 3 Gew.-%, bezogen auf die Einsatzstoffe. Bei Verwendung dieser Katalysatoren werden in erster Linie freie Hydroxylgruppen alkoxyliert.

Setzt man als Katalysatoren jedoch calcinierte oder mit Fettsäure hydrophobisierte Hydrotalcite ein, kommt es auch zu einer Insertion der Alkylenoxide in die Esterbindungen. Diese Methode ist bevorzugt, wenn man eine Alkylenoxidverteilung wünscht, die der bei Einsatz von alkoxylierten Di- oder Trialkanolaminen nahe kommt. Als Alkylenoxide können Ethylen- und Propylenoxid sowie deren Gemische (Random- oder Blockverteilung) eingesetzt werden. Die Reaktion wird üblicherweise bei Temperaturen im Bereich von 100 bis 180 °C durchgeführt. Durch den Einbau von im Durchschnitt 1 bis 10 Mol Alkylenoxid pro Mol Ester wird die Hydrophilie der Esterquats gesteigert, die Löslichkeit verbessert und die Reaktivität gegenüber anionischen Tensiden herabgesetzt.

Die Quaternierung der Ester kann in an sich bekannter Weise durchgeführt werden. Obschon die Umsetzung mit den Alkylierungsmitteln auch in Abwesenheit von Lösungsmitteln durchgeführt werden kann, empfiehlt sich die Mitverwendung zumindest von geringen Mengen Wasser oder kurzkettigen Alkoholen, vorzugsweise Isopropylalkohol, zur Herstellung von Konzentraten, die einen Feststoffanteil von mindestens 40 und insbesondere mindestens 60 Gew.-% aufweisen. In dieser Erfindung bedeutet der Begriff kurzkettige Alkohole eine C-Kettenlänge von C₁ - C₁₀.

Als Alkylierungsmittel kommen Alkylhalogenide wie beispielsweise Methylchlorid, Dialkylsulfate wie beispielsweise Dimethylsulfat oder Diethylsulfat oder Dialkylcarbonate wie beispielsweise Dimethylcarbonat oder Diethylcarbonat in Frage. Vorzugsweise ist die Alkylierung eine Methylierung oder eine Ethylierung, insbesondere eine Methylierung.

Üblicherweise werden die Ester und die Alkylierungsmittel im molaren Verhältnis - bezogen auf den Stickstoffgehalt des Esters - 1:0,50 bis 1:1,05 bevorzugt 1:0,90 bis 1:0,98 annähernd stöchiometrisch eingesetzt. Die Reaktionstemperatur liegt gewöhnlich bei 40 bis 110 °C und insbesondere bei 50 bis 80 °C. Im Anschluss an die Reaktion empfiehlt es sich, nicht umgesetztes Alkylierungsmittel durch Zugabe beispielsweise von Ammoniak, eine Alkanolamin, einer Aminosäure oder einem Oligopeptid zu zerstören, wie dies beispielsweise in DE-A1- 40 26 184 beschreiben wird.

In bestimmten Fällen kann es von Vorteil sein, die Eigenschaften der Esterquats durch Zugabe von definiertem Co-Kollektor, wie beispielsweise kationischen oder amphoteren Tensiden, zu modifizieren, anzupassen oder sogar zu unterstützen.

Kationische Tenside, die als Co-Kollektor verwendet werden können, sind insbesondere auszuwählen aus:
- primäre aliphatische Amine
- Alkylendiamine mit alpha-verzweigten Alkylreste
- hydroxyalkylsubstituiert Alkylendiamine,
- wasserlöslichen Säureadditionssalze dieser Amine,
- quartäre Ammoniumverbindungen, insbesondere
- quaternisiertes N,N-Dialkylaminoalkylamine.

Geeignete primäre aliphatische Amine sind vor allem C₈-C₂₂ Fettamine aus Fettsäuren von natürlichen Fetten und Ölen. Typische Bespiele sind n-Octylamin, n-Decylamin, n-Dodecylamin, n-Tetradecylamin, n-Hexadecylamin, n-Octadecylamine, n-Eicosylamin, n-Docosylamin, n-Hexadecenylamine und n-Octadecenylamin. Die genannten Amine können einzeln als Co-Kollektor verwendet werden, obwohl normalerweise Amingemische mit Alkyl- und/oder Alkenylreste aus dem Fettsäureanteil von tierischen oder pflanzlichen Fetten und Ölen verwendet werden.

Geeignete alkylsubstituierte Alkylendiamine für ihre Verwendung als Co-Kollektor entsprechen der Formel (IV), 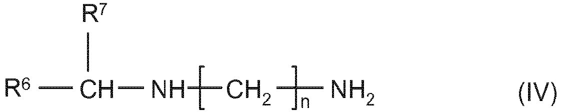 in der
- R⁶ und R⁷: für lineare oder verzweigte Alkyl- oder Alkenylreste steht und in der
- n: 2 bis 4 ist.

Die Herstellung dieser Verbindungen und ihre Verwendung bei der Flotation ist in DD 64275 beschrieben.

Geeignete hydroxyalkylsubstituierte Alkylendiamine für den Einsatz als Co-Kollektor entsprechen der Formel (V), 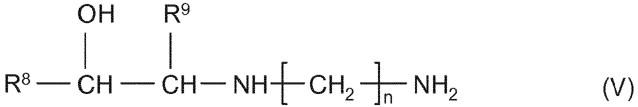 in der
- R⁸ und R⁹: für Wasserstoff und / oder lineare Alkylreste mit 1 bis 18 Kohlenstoffatome steht, die Summe der Kohlenstoffatome von R⁸ + R⁹ gleich 9 bis 18 ist, und
- n: 2 bis 4 ist.

Die Herstellung von Verbindungen der Formel (V) und ihre Verwendung bei der Flotation ist in DE-AS 2547987 beschrieben.

Die Aminverbindungen, die vorstehend erwähnt sind, können als solche oder in Form ihrer wasserlöslichen Salze verwendet werden. Die Salze werden in bestimmten Fällen durch Neutralisation mit äquimolaren Mengen, Über- oder Unterschuss an Säure erzielt. Geeignete Säuren sind beispielsweise Schwefelsäure, Phosphorsäure, Essigsäure und Ameisensäure.

Geeignete quartäre Ammoniumverbindungen zur Verwendung als Co-Kollektor entsprechen der Formel (VI), 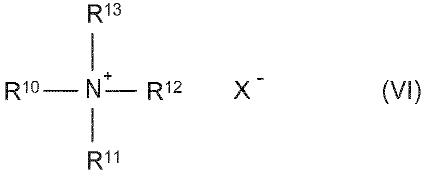 in denen
- R¹⁰: ein linearer Alkylrest mit 1 bis 18 Kohlenstoffatomen ist,
- R¹¹: ein Alkylrest mit 1 bis 18 Kohlenstoffatomen oder einen Benzylrest ist, und
- R¹² und R¹³: gleich oder verschieden sein können und jeweils für einen Alkylrest mit 1 bis 2 Kohlenstoffatomen stehen, und
- X: für ein Halogenid-Anion, insbesondere ein Chloridion steht.

Bevorzugt sind quartäre Ammoniumverbindungen, in denen R¹⁰ ein Alkylrest mit 8 bis 18 Kohlenstoffatomen ist und R¹¹, R¹² und R¹³ gleich sind und entweder Methyl- oder Ethylgruppen darstellen, und X ein Chloridion ist.

Die insbesondere bevorzugten kationischen Co-Kollektoren umfassen quaternisierte N,N-Dialkylaminoalkylamiden, die vorzugsweise der Formel (VII) entsprechen, 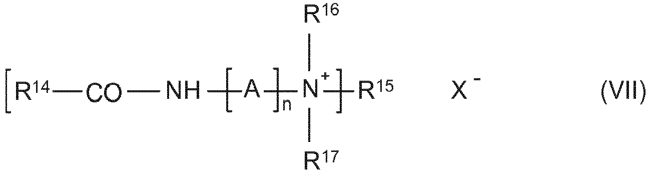 in der
- R¹⁴-CO: für einen aliphatischen, linearen oder verzweigten Acylrest mit 6 bis 22 Kohlenstoffatomen, vorzugsweise mit 12 bis 18 Kohlenstoffatomen steht und der 0, 1, 2 oder 3 Doppelbindungen enthält,
- [A]: für einen linearen oder verzweigten Alkylenrest mit 1 bis 4, vorzugsweise 2 oder 3, Kohlenstoffatomen steht,
- R¹⁵, R¹⁶ und R¹⁷: gleich oder verschieden sein können und jeweils einen Methyl- oder Ethylrest bedeuten, und
- X: ein Halogenid oder ein Alkylsulfat, insbesondere Methosulfat Anion ist.

Bevorzugt ist Kokosfettsäure-N,N-Dimethylaminopropylamid. Diese Produkte können, ebenfalls nach bekannter Weise, z. B. durch Umamidierung von N,N-Dimethylaminopropan mit hydrierten Kokosglyceriden und anschließende Quaternierung mittels Dimethylsulfat hergestellt werden. Vorzugsweise wird eine Mischung aus Kollektor und Co-Kollektor durch das Mischen der Ester und der N,N-Dialkylalkylamide und anschließend gemeinsame Quaternierung vorbereitet.

Gemäß der Erfindung enthalten die ampholytischen Tenside, die als Co-Kollektor verwendet werden können, mindestens eine anionische und eine kationische Gruppe im Molekül. Vorzugsweise sind die anionischen Gruppen Sulfonsäure- oder Carboxylgruppen und die kationischen Gruppen sind Aminogruppen, vorzugsweise sekundäre oder tertiäre Aminogruppen. Geeignete ampholytische Tenside sind insbesondere ausgewählt aus
- Sarkoside,
- Tauride,
- N-substituierten Aminopropionsäuren und
- N-(1,2-Dicarboxyethyl)-N-alkylsulfobernsteinsäureester.

Geeignete Sarkoside für ihre Verwendung als Co-Kollektor entsprechen der Formel (VIII) 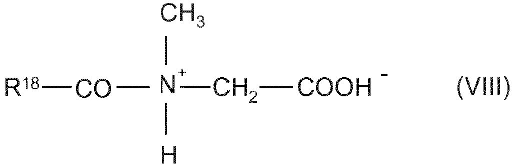 in denen
- R¹⁸: ein Alkylrest mit 7 bis 21 Kohlenstoffatomen, vorzugsweise 11 bis 17 Kohlenstoffatomen, ist.

Diese Sarkoside stellen bekannte Verbindungen dar, die nach bekannten Verfahren erhalten werden können. Ihre Verwendung bei der Flotation ist von H. Schubert in "Aufbereitung fester mineralischer Rohstoffe (Dressing fester mineralischer Rohstoffe)", 2. Auflage, Leipzig 1977, S. 310-311 und die Literaturangaben darin zitierten, beschrieben.

Geeignete Tauride für die Verwendung als Co-Kollektor entsprechen der Formel (IX) 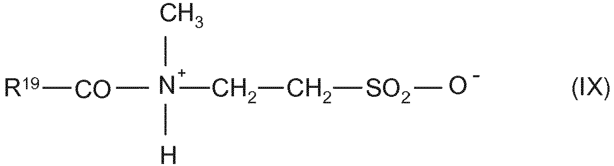 in der
- R¹⁹: ein Alkylrest mit 7 bis 21 Kohlenstoffatomen, vorzugsweise 11 bis 17 Kohlenstoffatomen, ist.

Diese Tauride stellen bekannte Verbindungen dar, die nach bekannten Verfahren erhalten werden können. Die Verwendung von Tauride bei der Flotation ist bekannt; vgl. H. Schubert, loc. cit.

Bevorzugte N-substituierte Aminopropionsäuren, geeignet zur Verwendung als Co-Kollektor entsprechen der Formel (X) 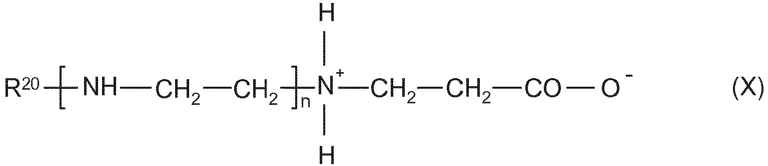 in der
- n: 0 oder eine Zahl von 1 bis 4 ist, und
- R²⁰: für einen Alkyl- oder Acylrest mit 8 bis 22 Kohlenstoffatomen steht.

Die genannten N-substituierten Aminopropionsäuren sind ebenfalls bekannte Verbindungen, die nach bekannten Methoden erhältlich sind. Ihre Verwendung als Kollektor bei der Flotation ist von H. Schubert, loc. cit. und in Int. J. Min. Proc. 9 (1982), Seiten 353 - 384 beschrieben.

Gemäß der Erfindung geeignete N-(1,2-Dicarboxyethyl)-N-alkylsulfobernsteinsäureester für die Verwendung als Co- Kollektor entsprechen der Formel (XI) 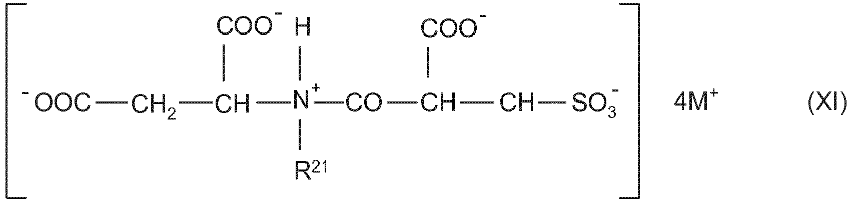 in der
- R²¹: für einen Alkylrest mit 8 bis 22 Kohlenstoffatomen, vorzugsweise 12 bis 18 Kohlenstoffatomen steht, und
- M: für ein Wasserstoffion, ein Alkalimetallkation oder ein Ammoniumion, vorzugsweise ein Natriumion steht.

Die erwähnten N-(1,2-Dicarboxyethyl)-N-alkylsulfobernsteinsäureester sind bekannte Verbindungen, die nach bekannten Verfahren erhalten werden können. Die Verwendung dieser Verbindungen als Kollektor bei der Flotation ist ebenfalls bekannt; vgl H. Schubert, loc. cit.

Vorzugsweise werden die erfindungsgemäßen Esterquats und die Co-Kollektoren in einem Gewichtsverhältnis von etwa 10:90 bis etwa 90:10, vorzugsweise etwa 25:75 bis etwa 75:25 und am meisten bevorzugt etwa 40:60 bis etwa 60:40 eingesetzt.

Zum Erreichen wirtschaftlich brauchbarer Ergebnisse bei der Flotation von nicht-sulfidischen Mineralien oder Erzen, müssen die Kollektoren bzw. die Mischungen von Kollektoren und Co-Kollektoren in einer bestimmten Mindestmenge verwendet werden. Jedoch sollte auch eine Höchstmenge an Kollektor / Co-Kollektor nicht überschritten werden, da es sonst zu übermäßig kräftigem Aufschäumen kommt und die Selektivität in Bezug auf die wertvollen Mineralien abnimmt.

Die Mengen, mit denen die Kollektoren erfindungsgemäß verwendet werden, sind durch die Art der Mineralien oder Erzen geregelt und sind abhängig vom anfänglichen Gehalt der wertvollen Mineralien. Dementsprechend kann die bestimmte Menge in weiten Grenzen variieren. Im Allgemeinen werden die Kollektoren und Kollektor / Co-Kollektor Mischungen gemäß der Erfindung in Mengen von 50 bis 2000 g / Tonne und vorzugsweise in Mengen von 100 bis 1500 g / Tonne Roherz eingesetzt.

Typische Schritte im Flotationsprozess sind in der Regel, zunächst die Trocken- oder vorzugsweise Nassmahlung der Mineralien oder Erze, die Suspension des resultierenden gemahlenen Minerals oder Erz in Wasser in Gegenwart der Flotationsreagenzien, und vorzugsweise nach einer Kontaktzeit der Flotationsreagenzien die Injektion von Luft in die Anlage. Im Folgenden werden die Natur der Ausgangsmaterialien sowie die Flotationshilfsmittel detaillierter veranschaulicht.

Flotierbare Mineralien und Erze können in die beiden Gruppen der polaren und nicht polaren Materialien unterteilt werden. Da die unpolaren Mineralien und Erze schwer zu hydratisieren sind, werden diese Materialien als hydrophob eingestuft. Beispiele für unpolare Mineralien sind Graphit, Molybdänit, Diamant, Kohle und Talk, die bereits in ihrem natürlich vorkommenden Zustand flotierbar sind. Im Gegensatz dazu haben polare Mineralien und Erze eine starke kovalente oder ionische Oberflächenbindung, die durch eine schnelle Hydrierung durch Wassermoleküle in Form von Multischichten zugänglich sind. Diese Ausgangsmaterialien, sind z. B. Calcit, Malachit, Azurit, Chrysokoll, Wulfenit, Cerussit, Witherit, Magnesit, Dolomit, Smithsonit, Rhodochrosit, Siderit, Magnetit, Monazit, Hämatit, Goethit, Chromit, Pyrolusit, Borax, Wolframit, Columbit, Tantalit, Rutil, Zirkon, Hemimorphit, Beryll, Glimmer, Biotit, Quarz, Feldspat, Kyanit und Granat. Die Flotation von nicht-sulfidischen, aber polaren Mineralien und Erzen ist ein bevorzugtes Ziel der vorliegenden Erfindung.

Das Flotationsverhalten der einzelnen mineralischen Bestandteile kann in gewissem Maß durch die Partikelgrößenverteilung des gemahlenen Minerals gesteuert werden. Umgekehrt kann jedoch auch die Partikelgröße die Wahl des Kollektors oder Kollektor / Co-Kollektor Gemischs beeinflussen. Im Allgemeinen kann jedoch gesagt werden, dass die Partikel mit zunehmender Partikelgröße auch mehr hydrophobisiert werden müssen, bevor die Partikel flotieren, was durch eine höhere Dosierrate bewirkt wird. Als allgemeine Regel gilt, dass die Erze so fein gemahlen sein müssen, dass die einzelnen feinen Teilchen nur aus einer Art von Mineral bestehen, nämlich entweder aus den wertvollen Mineralien oder den Verunreinigungen. Die ideale Korngröße muss normalerweise in Abhängigkeit vom speziellen Mineral bestimmt werden. Im vorliegenden Fall hat sich eine Partikelgrößenverteilung von etwa 5 bis 500 µm bewährt, wobei es in einigen Fällen praktikabler sein kann, eine schmalere Partikelgrößenverteilung zu wählen.

Beispielsweise können silikatreiche Erze hervorragend durch Flotation mit den Flotationskollektoren der vorliegenden Erfindung flotiert werden, wenn weniger als 40 Gew.-% (Gewichtsprozent), vorzugsweise 30 Gew.-%, besonders bevorzugt weniger als 15 Gew.-% des zu flotierenden Minerals oder Erzes kleiner sind als 250 µm. Um in der Lage zu sein eine optimale Flotation durchführen zu können, hat sich gezeigt, dass die Fraktion größer 125 µm kleiner als 15 Gew.-%, vorzugsweise weniger als 10 Gew.-% und am ehesten bevorzugt weniger als 5 Gew.-% des Minerals oder Erzes sein sollte. Die untere Grenze der Partikelgröße wird sowohl durch die Möglichkeit der mechanischen Größenreduzierung bestimmt, als auch durch die Eigenschaften der Mineralbestandteile bei der Flotation. In der Regel sollte der Anteil der Teilchen mit einem Durchmesser kleiner 50 µm bei 30 oder sogar 40 Gew.-% liegen. Gemäß der vorliegenden Erfindung ist es jedoch von besonderem Vorteil, wenn mehr als 40 Gew.-% der Teilchen kleiner 45 µm sind.

Zusätzliche Reagenzien, die die Oberflächenspannung oder Oberflächenchemie der Mineralien oder Erz verändern, können für die Flotation verwendet werden. Neben Kollektoren und Co-Kollektoren, die bereits oben erwähnt wurden, werden bei Bedarf zusätzlich Schäumer, pH-Regulatoren, Aktivatoren und Drücker eingesetzt.

In besonderen Fällen jedoch kann es notwendig oder zumindest vorteilhaft sein, je nach dem welches Flotationsverfahren verwendet wird, einen Schäumer einzusetzen. Die Zugabe von einem Schäumer ist dann notwendig, wenn die schäumende Eigenschaft des Kollektors oder des Kollektor/Co-Kollektor-Gemischs nicht ausreichend ist um eine angemessen hohe Schaumschicht zu produzieren, die während des Flotationsvorgangs stabil genug bleibt um die Mineralpartikel zu sammeln. Geeignete Schäumer sind aliphatische Alkohole, natürliche Öle, Glykole und Glykolether.

In besonderen Fällen jedoch kann es notwendig oder zumindest vorteilhaft sein, je nach dem welches Flotationsverfahren verwendet wird, einen Drücker einzusetzen. Die Zugabe von einem Drücker ist dann notwendig, wenn die Flotation von bestimmten Mineralien unterdrückt werden muss um das entsprechende Ausbringen und den Gehalt zu erreichen. Als Drücker werden beispielsweise natürliche Polysaccharide wie Guar, Stärke und Cellulose verwendet. Quebracho, Tannin, Dextrin sowie weitere chemische Derivate können auch verwendet werden, insbesondere die Derivate der Stärke, Guar und Cellulosemoleküle, deren Hydroxylgruppen mit einer breiten Palette an anionischen, kationischen und nicht-ionischen Funktionen ausgerüstet sein können.

Um das rheologische Verhalten der Kollektoren einzustellen, hilft bei der Flotation gemäß der vorliegenden Erfindung die Zugabe von Lösungsmittel in einer Menge von 5 bis 50 Gew.-%, vorzugsweise in einer Menge von 5 bis 40 Gew.-% und am meisten bevorzugt in einer Menge von 5 bis 30 Gew.-%. Geeignete Lösungsmittel sind beispielsweise aliphatische Alkohole mit kurzen Kettenlängen. Somit können die Flotationshilfsmittel gemäß der vorliegenden Erfindung geringe Mengen an Glykolen enthalten, beispielsweise Ethylenglykol, Propylenglykol oder Butylenglykol sowie einwertigen linearen oder verzweigten Alkoholen, beispielsweise Ethanol, n-Propanol oder Isopropanol.

Eine weitere Aufgabe der vorliegenden Erfindung ist die Verwendung von Esterquats als Kollektor für die Schaumflotation nicht-sulfidischer Mineralien oder Erzen. Die Kollektoren werden erfindungsgemäß bei der Flotation von nicht-sulfidischen Mineralien oder Erzen verwendet, die unter anderem Quarz, Kaolin, Glimmer, Phlogopit, Feldspat, Silikate und Eisenoxide enthalten.

### Beispiele

### Herstellungsbeispiel 1

528 g (1,9 Mol) teilweise gehärtete Palmölfettsäure, 212 g (1,1 Mol) Zitronensäure und 0,3 g Hypophosphorsäure wurden in einen Reaktor unter Rühren vorgelegt und bei vermindertem Druck von 20 mbar auf 120 °C erhitzt. Anschließend wurden portionsweise 447 g (3 mol) Triethanolamin zugegeben, wobei die Temperatur auf 130 °C anstieg. Nach Beendigung der Zugabe wurde der Ansatz auf 160 °C erhitzt, der Druck auf 3 mbar abgesenkt und die Mischung über einen Zeitraum von mindestens 10 h bei diesen Bedingungen gerührt, bis die Säurezahl auf einen Wert unterhalb von 5 mg KOH/g abgesunken war und eine typische Konsistenz erzielt wurde. Anschließend wurde die Mischung auf 60 °C abgekühlt, das Vakuum durch Einleiten von Stickstoff gebrochen und 0,6 g Wasserstoffperoxid in Form einer 30 gew.-%igen wässrigen Lösung zugegeben. Für die Quaternierung wurde der resultierende Ester in 376 g Isopropylalkohol gelöst und über einen Zeitraum von 1 h mit einer solchen Geschwindigkeit mit 357 g (2,83 mol) Dimethylsulfat (DMS) versetzt, dass die Temperatur nicht über 65 °C anstieg. Nach Beendigung der Zugabe ließ man den Ansatz weitere 2,5 h rühren, wobei der DMS-Restgehalt und Gesamtstickstoffgehalt durch Probenentnahme regelmäßig überprüft wurden. Die Reaktion wurde beendet, nachdem ein konstanter Gesamtstickstoffgehalt erreicht und kein DMS nachgewiesen wurde. Es wurde ein Produkt mit einem Feststoffgehalt von 80 Gew.-% erhalten.

### Herstellungsbeispiel 2

902 g (3,2 Mol) Ölsäure, 113 g (0,65 Mol) Aconitsäure und 0,9 g hypophosphorige Säure wurden in einen Reaktor unter Rühren vorgelegt und auf 100 °C unter einem verminderten Druck von 20 mbar erhitzt. Anschließend wurden portionsweise 447 g (3 mol) Triethanolamin zugetropft, wobei die Temperatur auf 120 °C anstieg. Nach Beendigung der Zugabe wurde der Ansatz auf 200 °C erhitzt, der Druck auf 3 mbar abgesenkt und die Mischung über einen Zeitraum von mindestens 6 h bei diesen Bedingungen gerührt, bis die Säurezahl auf einen Wert unterhalb von 5 mg KOH/g abgesunken war und eine typische Konsistenz erzielt wurde. Anschließend wurde die Mischung auf 60 °C abgekühlt, das Vakuum durch Einleiten von Stickstoff gebrochen und 0,6 g Wasserstoffperoxid in Form einer 30 gew.-%igen wässrigen Lösung zugegeben. Für die Quaternierung wurde der resultierende Ester in 736 g Isopropylalkohol gelöst und über einen Zeitraum von 1 h mit einer solchen Geschwindigkeit mit 357 g (2,83 mol) Dimethylsulfat versetzt, dass die Temperatur nicht über 65 °C anstieg. Nach Beendigung der Zugabe ließ man den Ansatz weitere 2,5 h rühren, wobei der DMS-Restgehalt und Gesamtstickstoffgehalt durch Probenentnahme regelmäßig überprüft wurden. Die Reaktion wurde beendet, nachdem ein konstanter Gesamtstickstoffgehalt erreicht und kein DMS nachgewiesen wurde. Es wurde ein Produkt mit einem Feststoffgehalt von 70 Gew.-% erhalten.

### Herstellungsbeispiel 3

590 g (2,1 Mol) destillierte Mischfettsäure (hauptsächlich C₁₆-C₂₀), 230 g (1,2 Mol) Zitronensäure wurden in einen Reaktor unter Rühren vorgelegt und auf max. 120 °C erhitzt. Anschließend wurden portionsweise 447 g (3 mol) Triethanolamin zugetropft, wobei die Temperatur auf max. 130 °C anstieg. Nach Beendigung der Zugabe wurde der Ansatz auf 180 °C erhitzt und die Mischung über einen Zeitraum von mindestens 10 h bei diesen Bedingungen gerührt, bis die Säurezahl auf einen Wert unterhalb von 5 mg KOH/g abgesunken war und eine typische Konsistenz erzielt wurde. Für die Quaternierung wurde der resultierende Ester in 610 g Isopropylalkohol gelöst und über einen Zeitraum von 1 h mit einer solchen Geschwindigkeit mit 357 g (2,83 mol) Dimethylsulfat versetzt, dass die Temperatur nicht über 65 °C anstieg. Nach Beendigung der Zugabe ließ man den Ansatz weitere 2,5 h rühren, wobei der DMS-Restgehalt und Gesamtstickstoffgehalt durch Probenentnahme regelmäßig überprüft wurden. Die Reaktion wurde beendet, nachdem ein konstanter Gesamtstickstoffgehalt erreicht und kein DMS nachgewiesen wurde. Es wurde ein Produkt mit einem Feststoffgehalt von 70 Gew.-% erhalten.

### Herstellungsbeispiel 4

28 g (0,2 mol) Caprylsäure, 23 g (0,12 Mol) Zitronensäure wurden in einen Reaktor unter Rühren vorgelegt und auf max. 120 °C erhitzt. Anschließend wurden portionsweise 192 g (0,45 mol) Genamin C050 (ein C₁₂/C₁₄-Amin mit 5 mol Ethylenoxid) zugetropft, wobei die Temperatur auf max 130 °C anstieg. Nach Beendigung der Zugabe wurde der Ansatz auf 180 °C erhitzt und die Mischung über einen Zeitraum von mindestens 10 h bei diesen Bedingungen gerührt, bis die Säurezahl auf einen Wert unterhalb von 5 mg KOH/g abgesunken war und eine typische Konsistenz erzielt wurde. Für die Quaternierung wurde der resultierende Ester in 286 g Isopropylalkohol gelöst und über einen Zeitraum von 1 h mit einer solchen Geschwindigkeit mit 53,7 g (0,43 mol) Dimethylsulfat versetzt, dass die Temperatur nicht über 65 °C anstieg. Nach Beendigung der Zugabe ließ man den Ansatz weitere 2,5 h rühren, wobei der DMS-Restgehalt und Gesamtstickstoffgehalt durch Probenentnahme regelmäßig überprüft wurden. Die Reaktion wurde beendet, nachdem ein konstanter Gesamtstickstoffgehalt erreicht und kein DMS nachgewiesen wurde. Es wurde ein Produkt mit einem Feststoffgehalt von 50 Gew.-% erhalten

### Vergleichsbeispiel [WO2008089906]

In einem Rührreaktor wurden 567 g (2,1 mol) teilgehartete Palmfettsaure, 219 g (1,5 mol) Adipinsäure und 0,3 g Hypophosphorsäure Hypophosphorsäure in einen Reaktor unter Rühren vorgelegt und bei vermindertem Druck von 20 mbar auf 70 °C erhitzt. Anschließend wurden portionsweise 447 g (3 mol) Triethanolamin, wobei die Temperatur auf max. 120 °C anstieg. Nach Beendigung der Zugabe wurde der Ansatz auf 160 °C erhitzt, der Druck auf 3 mbar abgesenkt und die Mischung über einen Zeitraum von 2,5 h bei diesen Bedingungen gerührt, bis die Säurezahl auf einen Wert unterhalb von 5 mg KOH/g abgesunken war.

Anschließend wurde die Mischung auf 60 °C abgekühlt, das Vakuum durch Einleiten von Stickstoff gebrochen und 0,6 g Wasserstoffperoxid in Form einer 30 gew.-%igen wässrigen Lösung zugegeben. Für die Quaternierung wurde der resultierende Ester in 376 g Isopropylalkohol gelöst und über einen Zeitraum von 1 h mit einer solchen Geschwindigkeit mit 357 g (2,83 mol) Dimethylsulfat (DMS) versetzt, dass die Temperatur nicht über 65 °C anstieg. Nach Beendigung der Zugabe ließ man den Ansatz weitere 2,5 h rühren, wobei der DMS-Restgehalt und

Gesamtstickstoffgehalt durch Probenentnahme regelmäßig überprüft wurden. Die Reaktion wurde beendet, nachdem ein konstanter Gesamtstickstoffgehalt erreicht und kein DMS nachgewiesen wurde. Es wurde ein Produkt mit einem Feststoffgehalt von 80 Gew.-% erhalten.

### Anwendungsbeispiele

Die folgenden Beispiele zeigen die überlegene Wirksamkeit der neuen Esterquats der Erfindung gegenüber Kollektorkomponenten aus dem Stand der Technik aus WO-2008/089906, insbesondere im Vergleich zu konventionellen polymeren Esterquats, die mit Mono- und Disäuren synthetisiert wurden. Die Tests wurden unter Laborbedingungen durchgeführt und dementsprechend können sich die gewählten Parameter von der Praxis unterscheiden. Die angegebenen Mengen der Reagenzien beziehen sich auf die Aktivsubstanz.

Die Flotation unter Laborbedingungen wurde gemäß Stand der Technik durchgeführt. Die Trennung der säureunlöslichen Mineralien, bei denen es sich um Silikate handelt, von Calcit durch umgekehrte Flotation erfolgt durch Zuführen des gemahlenen Ausgangsstoffs zu einer Flotationszelle. In der Flotationszelle wird der Ausgangsstoff in Wasser suspendiert. Nach Zugabe eines Silikatkollektors und Verstreichen einer Konditionierungszeit beginnt die Flotation. Die Silikate sammeln sich in der Schaumphase, das Calcit bleibt in der Flotationszelle.

Die folgenden Beispiele veranschaulichen die Wirksamkeit der Kollektoren der vorliegenden Erfindung im Vergleich zu herkömmlichen polymeren Esterquats, die mit Mono- und Disäuren synthetisiert wurden, bei der Flotation von silikathaltigen Calcit-Mineralien. Die Ergebnisse sind in Tabelle 1 gezeigt.

| | |
|---|---|
| Korngrößenverteilung: | 60 Gew.-% aller Partikel > 45 µm |
| Säureunlösliches | 2,8 bis 3,5 Gew.-% |
| Calcit: | ca. 96,5 - 97,2 Gew.-% |

**Tabelle 1: Testergebnisse Calcit Flotation**

| Testergebnisse | Dosierung g/t | Masse Berge | Masse Konzentrat | Konzentratgehalt HCl-Unlösliches | Calcit Verlust |
|---|---|---|---|---|---|
| | (Aktivgehalt) | [g] | [g] | [%] | [%] |
| Vergleichsbeispiel | 510 | 268.8 | 1447.2 | 0.01 | 13.9 |
| | 425 | 204.3 | 1511.7 | 0.03 | 10.1 |
| | 310 | 128.7 | 1587.3 | 0.07 | 5.6 |
| Herstellungsbeispiel 1 | 510 | 106.1 | 1609.9 | 0.01 | 8.7 |
| | 425 | 73.2 | 1642.8 | 0.05 | 2.4 |
| | 310 | 58.8 | 1657.3 | 0.13 | 1.6 |
| Herstellungsbeispiel 2 | 425 | 178.5 | 1537.5 | 0.01 | 8.6 |
| | 320 | 110.9 | 1605.1 | 0.04 | 4.6 |
| Herstellungsbeispiel 3 | 370 | 189.0 | 1527.0 | 0.01 | 9.2 |
| | 280 | 133.9 | 1582.1 | 0.01 | 5.9 |
| | 210 | 68.5 | 1647.6 | 0.05 | 2.5 |
| Herstellungsbeispiel 4 | 645 | 136.3 | 1579.7 | 0.02 | 6.1 |
| | 510 | 106.1 | 1609.9 | 0.02 | 4.3 |
| | 325 | 65.4 | 1650.6 | 0.09 | 1.9 |

Der Calcitverlust stellt die Menge an Calcit dar, die bei der reversen Flotation zusammen mit Silikaten, Eisenoxiden und anderen Verunreinigungen ausgetragen werden. Der Gehalt an HCl-Unlöslichem im Konzentrat steht für die nach im gereinigten Calcit vorhandenen Verunreinigungen an Silikaten, Eisenoxiden etc. Ziel ist es, dass sowohl Calcitverlust als auch HCl-Unlösliches im Konzentrat möglichst gering sind.

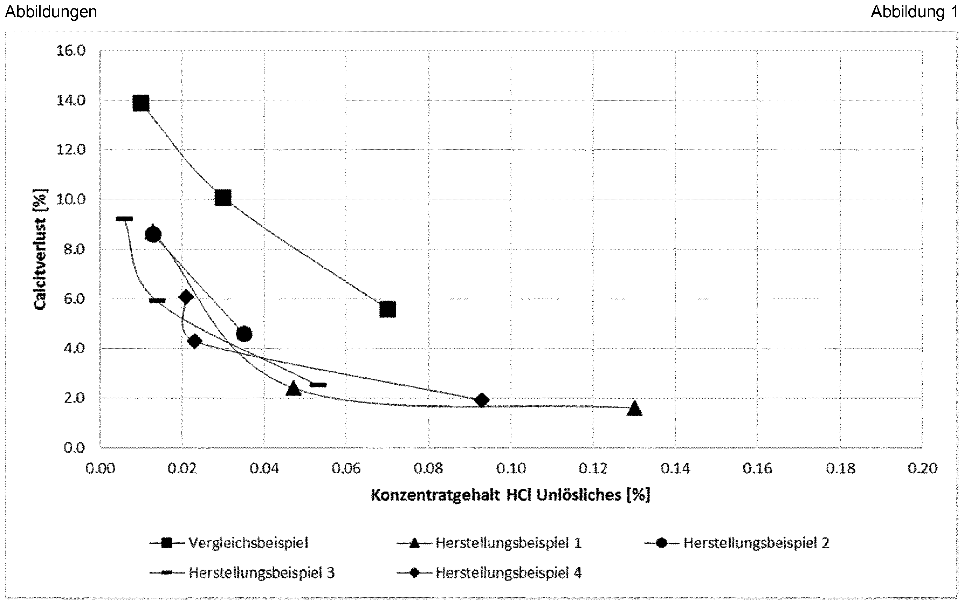

Die Produkte der Herstellungsbeispiele 1 - 4 erlauben im Vergleich mit dem Vergleichsbeispiel eine verbesserte reverse Calcitflotation. Dies wird graphisch auch in Abbildung 1 dargestellt.

## Patentansprüche

1. Esterquats, dadurch erhältlich, indem man Di- oder Trialkanolamine mit einer Mischung aus Fettsäuren und Polycarbonsäuren umsetzt, und die so erhaltenen Ester anschließend mit einem Alkylierungsmittel quaterniert, wobei die Di- oder Trialkanolamine der Formel (I) entsprechen 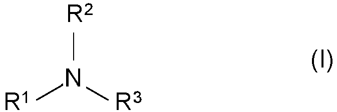 worin
R¹ und R² unabhängig voneinander für Hydroxyalkylreste mit 1 bis 20 Kohlenstoffatomen, Hydroxyalkenylreste mit 2 bis 20 Kohlenstoffatomen und 1, 2, oder 3 Doppelbindungen, oder Anlagerungsprodukte von 1 bis 20 Mol Ethylenoxid an einen Hydroxyethylrest stehen, und
R³ für Wasserstoff, einen Alkylrest mit 1 bis 20 Kohlenstoffatomen, einen Alkenylrest mit 2 bis 20 Kohlenstoffatomen und 1, 2, oder 3 Doppelbindungen, einen Hydroxyalkylrest mit 1 bis 20 Kohlenstoffatomen, einen Hydroxyalkenylrest mit 2 bis 20 Kohlenstoffatomen und 1, 2, oder 3 Doppelbindungen, oder Anlagerungsprodukte von 1 bis 20 Mol Ethylenoxid an einen Hydroxyethylrest steht, und wobei
die Fettsäuren der Formel (II) entsprechen,
R⁴-COOH (II)
worin
R⁴ für einen aliphatischen, linearen oder verzweigten Kohlenwasserstoffrest mit 5 bis 29 Kohlenstoffatomen und 0, 1, 2 oder 3 Doppelbindungen steht, **dadurch gekennzeichnet, dass**
die Polycarbonsäuren der Formel (III) entsprechen 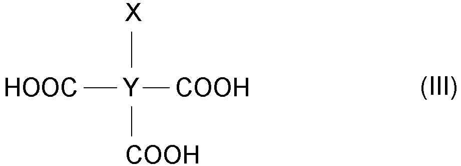 worin
Y für ein Kohlenstoffatom oder eine gesättigte oder ungesättigte, aliphatische Kohlenwasserstoffgruppe mit 2 bis 5 Kohlenstoffatomen steht, welche gegebenenfalls eine oder mehrere Hydroxylgruppen trägt, und
X für Wasserstoff, eine Hydroxy-, oder eine Carbonsäuregruppe stehen.

2. Esterquats nach Anspruch 1, **dadurch gekennzeichnet, dass** die Di- oder Trialkanolamine der Formel (I) als Reste R¹ und R² unabhängig voneinander Hydroxyalkylreste mit 2 bis 5 Kohlenstoffatomen oder Hydroxyalkenylreste mit 2 bis 5 Kohlenstoffatomen und 1, 2 oder 3 Doppelbindungen, oder Anlagerungsprodukte von 2 bis 5 Mol Ethylenoxid an einen Hydroxyethylrest enthhalten

3. Esterquats nach Anspruch 1 und/oder 2, **dadurch gekennzeichnet, dass** R³ für Alkylreste mit 1 bis 20 Kohlenstoffatomen, Alkenylreste mit 2 bis 20 Kohlenstoffatomen, Hydroxyalkylreste mit 2 bis 5 Kohlenstoffatomen oder Hydroxyalkenylreste mit 2 bis 5 Kohlenstoffatomen und 1, 2, oder 3 Doppelbindungen steht.

4. Esterquats nach Anspruch 1, **dadurch gekennzeichnet, dass** die Di- oder Trialkanolamine der Formel (I) aus der Gruppe bestehend aus Methyldiethanolamin (MDA), Diethanolamin (DEA), diethoxylierte Oleylamine und Triethanolamin (TEA) ausgewählt sind.

5. Esterquats nach einem oder mehreren der Ansprüche 1 bis 4, **dadurch gekennzeichnet dass** in den Fettsäuren der Formel (II) R⁴ für einen aliphatischen, linearen oder verzweigten Rest mit 7 bis 21 Kohlenstoffatomen und 0, 1, 2 oder 3 Doppelbindungen steht.

6. Esterquats nach einem oder mehreren der Ansprüche 1 bis 4, **dadurch gekennzeichnet dass** die Fettsäuren der Formel (II) ausgewählt sind aus der Gruppe bestehend aus Caprylsäure, , Caprinsäure, Laurinsäure, Undecylensäure, Isotridecansäure, Myristinsäure, Myristoleinsäure, Palmitinsäure, Palmitoleinsäure, Stearinsäure, Isostearinsäure, Ölsäure, Petroselinsäure, Elaidinsäure, Linolsäure, Linolensäure, Elaeostearinsäure, Arachinsäure, Gadoleinsäure, Behensäure, Erucasäure und Cetoleinsäure sowie deren technische Mischungen.

7. Esterquats nach einem oder mehreren der Ansprüche 1 bis 6, **dadurch gekennzeichnet dass** die Polycarbonsäure der Formel (III) als Rest Y eine gesättigte oder ungesättigte aliphatische Kohlenwasserstoffgruppe mit 3 oder 4 Kohlenstoffatomen, welche gegebenenfalls eine oder mehrere Hydroxylgruppen trägt, umfasst.

8. Esterquats nach einem oder mehreren der Ansprüche 1 bis 6, **dadurch gekennzeichnet dass** die Polycarbonsäure aus der Gruppe bestehend aus Propan-1,2,3-tricarbonsäure, Aconitsäure, Isozitronensäure und insbesondere Zitronensäure ausgewählt ist.

9. Esterquats nach einem oder mehreren der Ansprüche 1 bis 8, **dadurch gekennzeichnet dass** die Fettsäuren und die Polycarbonsäuren im molaren Verhältnis von 1:10 bis 10:1 eingesetzt werden.

10. Esterquats nach einem oder mehreren der Ansprüche 1 bis 9, **dadurch gekennzeichnet dass** die Di- oder Trialkanolamine einerseits und die Gesamtmenge der Fettsäuren und Polycarbonsäuren anderseits im molaren Verhältnis von 1:0,25 bis 1:3,0 eingesetzt werden.

11. Esterquats nach einem oder mehreren der Ansprüche 1 bis 9, **dadurch gekennzeichnet dass** ein Alkylierungsmittel ausgewählt aus der Gruppe bestehend aus Alkylhalogeniden, Dialkylsulfaten und Dialkylcarbonaten eingesetzt wird.

12. Esterquats nach Anspruch 11, **dadurch gekennzeichnet, dass** Alkyl für Methyl oder Ethyl steht.

13. Verwendung von Esterquats nach einem oder mehreren der Ansprüche 1 bis 12 als Kollektor für die Schaumflotation von nicht-sulfidischen Mineralien und Erzen, **dadurch gekennzeichnet, dass** das Erz ein Calcitmineral ist.

14. Verfahren zur Flotation von Calcitmineral, bei dem das Calcitmineral mit Wasser und einem Esterquat nach einem oder mehreren der Ansprüche 1 bis 12 gemischt werden um eine Suspension zu bilden, in die Suspension Luft eingeführt und der aufgeschwemmte Schaum entfernt wird

15. Verfahren nach Anspruch 14, **dadurch gekennzeichnet, dass** quaternisierte N,N- Dialkylaminoalkylamide der Formel (VII), 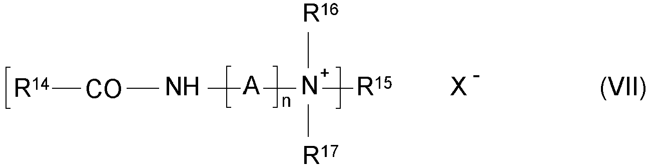 in der
R¹⁴-CO für einen aliphatischen, linearen oder verzweigten Acylrest mit 6 bis 22 Kohlenstoffatomen steht und der 0, 1, 2 oder 3 Doppelbindungen enthält,
[A] für einen linearen oder verzweigten Alkylenrest mit 1 bis 4 Kohlenstoffatomen steht,
R¹⁵, R¹⁶ und R¹⁷ gleich oder verschieden sein können und jeweils einen Methyl- oder Ethylrest bedeuten, und
X ein Halogenid oder ein Alkylsulfat Anion ist,
als Co-Sammler eingesetzt werden.

16. Verfahren nach Anspruch 15, **dadurch gekennzeichnet, dass** das Esterquat nach einem oder mehreren der Ansprüche 1 bis 12 und der Co-Kollektor gemäß Formel (VII) in einem Gewichtsverhältnis von 10:90 bis 90:10 verwendet werden.

## Claims

1. Esterquats obtainable by reacting di- or trialkanolamines with a mixture of fatty acids and polycarboxylic acids and quaternizing the resultant esters subsequently with an alkylating agent, where the di- or trialkanolamines conform to the formula (I) 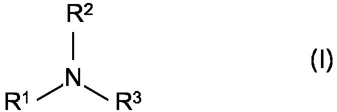 in which
R¹ and R² independently of one another are hydroxyalkyl radicals having 1 to 20 carbon atoms, hydroxyalkenyl radicals having 2 to 20 carbon atoms and 1, 2, or 3 double bonds, or adducts of 1 to 20 mol of ethylene oxide onto a hydroxyethyl radical, and
R³ is hydrogen, an alkyl radical having 1 to 20 carbon atoms, an alkenyl radical having 2 to 20 carbon atoms and 1, 2, or 3 double bonds, a hydroxyalkyl radical having 1 to 20 carbon atoms, a hydroxyalkenyl radical having 2 to 20 carbon atoms and 1, 2, or 3 double bonds, or adducts of 1 to 20 mol of ethylene oxide onto a hydroxyethyl radical, and where
the fatty acids conform to the formula (II),
R⁴-COOH (II)
in which
R⁴ is an aliphatic, linear or branched hydrocarbon radical having 5 to 29 carbon atoms and 0, 1, 2, or 3 double bonds, **characterized in that**
the polycarboxylic acids conform to the formula (III) 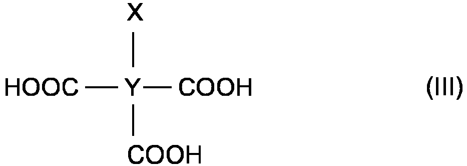 in which
Y is a carbon atom or a saturated or unsaturated, aliphatic hydrocarbon group having 2 to 5 carbon atoms and optionally carrying one or more hydroxyl groups, and
X is hydrogen, a hydroxyl or a carboxylic acid group.

2. Esterquats according to Claim 1, **characterized in that** the di- or trialkanolamines of the formula (I) comprise as radicals R¹ and R², independently of one another, hydroxyalkyl radicals having 2 to 5 carbon atoms or hydroxyalkenyl radicals having 2 to 5 carbon atoms and 1, 2, or 3 double bonds, or adducts of 2 to 5 mol of ethylene oxide onto a hydroxyethyl radical.

3. Esterquats according to Claim 1 and/or 2, **characterized in that** R³ represents alkyl radicals having 1 to 20 carbon atoms, alkenyl radicals having 2 to 20 carbon atoms, hydroxyalkyl radicals having 2 to 5 carbon atoms, or hydroxyalkenyl radicals having 2 to 5 carbon atoms and 1, 2, or 3 double bonds.

4. Esterquats according to Claim 1, **characterized in that** the di- or trialkanolamines of the formula (I) are selected from the group consisting of methyldiethanolamine (MDA), diethanolamine (DEA), diethoxylated oleylamines, and triethanolamine (TEA).

5. Esterquats according to one or more of Claims 1 to 4, **characterized in that** in the fatty acids of the formula (II), R⁴ is an aliphatic, linear or branched radical having 7 to 21 carbon atoms and 0, 1, 2, or 3 double bonds.

6. Esterquats according to one or more of Claims 1 to 4, **characterized in that** the fatty acids of the formula (II) are selected from the group consisting of caprylic acid, capric acid, lauric acid, undecylenic acid, isotridecanoic acid, myristic acid, myristoleic acid, palmitic acid, palmitoleic acid, stearic acid, isostearic acid, oleic acid, petroselinic acid, elaidic acid, linoleic acid, linolenic acid, eleostearic acid, arachidic acid, gadoleic acid, behenic acid, erucic acid, and cetoleic acid, and also technical mixtures thereof.

7. Esterquats according to one or more of Claims 1 to 6, **characterized in that** the polycarboxylic acid of the formula (III) comprises as radical Y a saturated or unsaturated aliphatic hydrocarbon group having 3 or 4 carbon atoms and optionally carrying one or more hydroxyl groups.

8. Esterquats according to one or more of Claims 1 to 6, **characterized in that** the polycarboxylic acid is selected from the group consisting of propane-1,2,3-tricarboxylic acid, aconitic acid, isocitric acid and, in particular, citric acid.

9. Esterquats according to one or more of Claims 1 to 8, **characterized in that** the fatty acids and the polycarboxylic acids are used in a molar ratio of 1:10 to 10:1.

10. Esterquats according to one or more of Claims 1 to 9, **characterized in that** the di- or trialkanolamines on the one hand and the total amount of the fatty acids and polycarboxylic acids on the other hand are used in a molar ratio of 1:0.25 to 1:3.0.

11. Esterquats according to one or more of Claims 1 to 9, **characterized in that** an alkylating agent selected from the group consisting of alkyl halides, dialkyl sulfates, and dialkyl carbonates is used.

12. Esterquats according to Claim 11, **characterized in that** alkyl is methyl or ethyl.

13. Use of esterquats according to one or more of Claims 1 to 12 as collectors for the froth flotation of non-sulfidic minerals and ores, **characterized in that** the ore is a calcite mineral.

14. Method for flotation of calcite mineral, wherein the calcite mineral is mixed with water and an esterquat according to one or more of Claims 1 to 12 to form a suspension, air is introduced into the suspension, and the floated froth is removed.

15. Method according to Claim 14, **characterized in that** quaternized N,N-dialkylaminoalkylamides of the formula (VII), 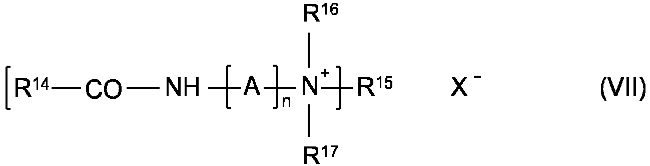 in which
R¹⁴-CO is an aliphatic, linear or branched acyl radical having 6 to 22 carbon atoms and containing 0, 1, 2, or 3 double bonds,
[A] is a linear or branched alkylene radical having 1 to 4 carbon atoms,
R¹⁵, R¹⁶ and R¹⁷ may be identical or different and each denote a methyl or ethyl radical, and
X is a halide or an alkylsulfate anion,
are used as co-collectors.

16. Method according to Claim 15, **characterized in that** the esterquat according to one or more of Claims 1 to 12 and the co-collector of formula (VII) are used in a weight ratio of 10:90 to 90:10.

## Revendications

1. Esterquats, pouvant être obtenus par réaction de di- ou de trialcanolamines avec un mélange d'acides gras et d'acides polycarboxyliques, et les esters ainsi obtenus étant ensuite quaternisés avec un agent d'alkylation, les di- ou trialcanolamines correspondant à la Formule (I) 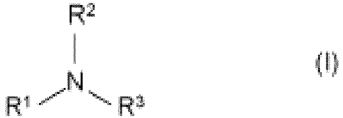 dans laquelle
R¹ et R² représentent indépendamment l'un de l'autre des radicaux hydroxyalkyle ayant 1 à 20 atomes de carbone, des radicaux hydroxyalcényle ayant 2 à 20 atomes de carbone et 1, 2 ou 3 doubles liaisons, ou des produits d'addition de 1 à 20 moles d'oxyde d'éthylène sur un radical hydroxyéthyle, et
R³ représente un hydrogène, un radical alkyle ayant 1 à 20 atomes de carbone, un radical alcényle ayant 2 à 20 atomes de carbone et 1, 2 ou 3 doubles liaisons, un radical hydroxyalkyle ayant 1 à 20 atomes de carbone, un radical hydroxyalcényle ayant 2 à 20 atomes de carbone et 1, 2 ou 3 doubles liaisons, ou des produits d'addition de 1 à 20 moles d'oxyde d'éthylène sur un radical hydroxyéthyle, et
les acides gras correspondant à la Formule (II)
R⁴-COOH (II)
dans laquelle
R⁴ représente un radical hydrocarboné aliphatique, linéaire ou ramifié, ayant 5 à 29 atomes de carbone et 0, 1, 2 ou 3 doubles liaisons, **caractérisés en ce que**
les acides polycarboxyliques correspondent à la Formule (III) 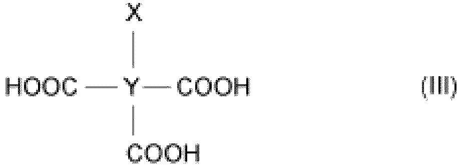 dans laquelle
Y représente un atome de carbone ou un groupe hydrocarboné aliphatique saturé ou insaturé ayant 2 à 5 atomes de carbone, qui éventuellement porte un ou plusieurs groupes hydroxyle, et
X représente un hydrogène, un groupe hydroxy ou acide carboxylique.

2. Esterquats selon la revendication 1, **caractérisés en ce que** les di- ou trialcanolamines de Formule (I) contiennent en tant que radicaux R¹ et R², indépendamment l'un de l'autre, des radicaux hydroxyalkyle ayant 2 à 5 atomes de carbone ou des radicaux hydroxyalcényle ayant 2 à 5 atomes de carbone et 1, 2 ou 3 doubles liaisons, ou des produits d'addition de 2 à 5 moles d'oxyde d'éthylène sur un radical hydroxyéthyle.

3. Esterquats selon les revendications 1 et/ou 2, **caractérisés en ce que** R³ représente des radicaux alkyle ayant 1 à 20 atomes de carbone, des radicaux alcényle ayant 2 à 20 atomes de carbone, des radicaux hydroxyalkyle ayant 2 à 5 atomes de carbone ou des radicaux hydroxyalcényle ayant 2 à 5 atomes de carbone et 1, 2 ou 3 doubles liaisons.

4. Esterquats selon la revendication 1, **caractérisés en ce que** les di- ou trialcanolamines de Formule (I) sont choisies dans le groupe consistant la méthyldiéthanolamine (MDA), la diéthanolamine (DEA), les oléylamines diéthoxylées et la triéthanolamine (TEA).

5. Esterquats selon l'une ou plusieurs des revendications 1 à 4, **caractérisés en ce que**, dans les acides gras de Formule (II), R⁴ représente un radical aliphatique, linéaire ou ramifié ayant 7 à 21 atomes de carbone et 0, 1, 2 ou 3 doubles liaisons.

6. Esterquats selon l'une ou plusieurs des revendications 1 à 4, **caractérisés en ce que** les acides gras de Formule (II) sont choisis dans le groupe consistant en l'acide caprylique, l'acide caprique, l'acide laurique, l'acide undécylénoïque, l'acide isotridécanoïque, l'acide myristique, l'acide myristoléique, l'acide palmitique, l'acide palmitoléique, l'acide stéarique, l'acide isostéarique, l'acide oléique, l'acide pétrosélinique, l'acide élaïdique, l'acide linoléique, l'acide linolénique, l'acide élaéostéarique, l'acide arachidique, l'acide gadoléique, l'acide béhénique, l'acide érucasique et l'acide cétoléique, ainsi que leurs mélanges techniques.

7. Esterquats selon l'une ou plusieurs des revendications 1 à 6, **caractérisés en ce que** l'acide polycarboxylique de Formule (III) comprend en tant que radical Y un groupe hydrocarboné aliphatique saturé ou insaturé ayant 3 ou 4 atomes de carbone, qui éventuellement porte un ou plusieurs groupes hydroxyle.

8. Esterquats selon l'une ou plusieurs des revendications 1 à 6, **caractérisés en ce que** l'acide polycarboxylique est choisi dans le groupe consistant en l'acide propane-1,2,3-tricarboxylique, l'acide aconitique, l'acide isocitrique et en particulier l'acide citrique.

9. Esterquats selon l'une ou plusieurs des revendications 1 à 8, **caractérisés en ce que** les acides gras et les acides polycarboxyliques sont utilisés selon un rapport en moles de 1:10 à 10:1.

10. Esterquats selon l'une ou plusieurs des revendications 1 à 9, **caractérisés en ce que** les di- ou trialcanolamines d'une part, et la quantité totale des acides gras et des acides polycarboxyliques d'autre part, sont utilisées selon un rapport en moles de 1:0,25 à 1:3,0.

11. Esterquats selon l'une ou plusieurs des revendications 1 à 9, **caractérisés en ce qu'**on utilise un agent d'alkylation choisi dans le groupe consistant en les halogénures d'alkyle, les sulfates de dialkyle et les carbonates de dialkyle.

12. Esterquats selon la revendication 11, **caractérisés en ce qu'**alkyle représente un méthyle ou un éthyle.

13. Utilisation d'esterquats selon l'une ou plusieurs des revendications 1 à 12 en tant que collecteur pour la flottation de mousse de minéraux et de minerais non sulfurés, **caractérisée en ce que** le minerai est un minéral calcite.

14. Procédé de flottation d'un minéral calcite, dans lequel le minéral calcite est mélangé à de l'eau et à un esterquat selon l'une ou plusieurs des revendications 1 à 12 pour former une suspension, on introduit de l'air dans la suspension et on élimine la mousse qui flotte.

15. Procédé selon la revendication 14, **caractérisé en ce qu'**on utilise comme co-collecteur des N,N-dialkylaminoalkylamides quaternisés de Formule (VII), 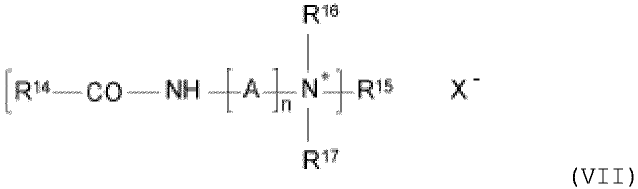 dans laquelle
R¹⁴-CO représente un radical acyle aliphatique, linéaire ou ramifié ayant 6 à 22 atomes de carbone, et qui contient 0, 1, 2 ou 3 doubles liaisons,
[A] représente un radical alkylène linéaire ou ramifié ayant 1 à 4 atomes de carbone,
R¹⁵, R¹⁶ et R¹⁷ peuvent être identiques ou différents et représentent chacun un radical méthyle ou éthyle, et
X représente un anion halogénure ou alkylsulfate.

16. Procédé selon la revendication 15, **caractérisé en ce que** l'esterquat selon l'une ou plusieurs des revendications 1 à 12 et le co-collecteur selon la Formule (VII) sont utilisés selon un rapport en poids de 10:90 à 90:10.
